# EUROPÄISCHE PATENTANMELDUNG

(11) **EP 4 257 670 A1**
(43) Veröffentlichungstag der Anmeldung: **11.10.2023**
(21) Anmeldenummer: 23163109.4
(22) Anmeldetag: 21.03.2023
(51) Int. Cl.: C12M 3/00, A61L 27/38, C12M 1/12, C12M 1/26, C12N 5/071

(54) **VERFAHREN ZUM DREIDIMENSIONALEN NACHBILDEN EINES BIOLOGISCHEN GEWEBES**

(30) Priorität: 04.04.2022 DE 102022108006
(71) Anmelder: Technische Universität Ilmenau, 98693 Ilmenau (DE)
(72) Erfinder: SCHOBER, Andreas, 99096 Erfurt (DE); WEISE, Frank, 98693 Ilmenau (DE); HAMPL, Jörg, 99090 Erfurt (DE); BRAUER, Dana, 98716 Elgersburg (DE)
(74) Vertreter: Rittermann, Marco

(57) **Zusammenfassung**

Die vorliegende Erfindung betrifft ein Verfahren zum dreidimensionalen Nachbilden eines biologischen Gewebes im Rahmen des Tissue Engineering. Gemäß diesem Verfahren wird ein Substrat (01) bereitgestellt und es werden primäre Zellen (08) eines ersten Zelltyps auf dem Substrat (01) vorgelegt. Ein erster Präkursor einer die Zellen (08) verklebenden Substanz (11) wird auf zumindest einer Auswahl der primären Zellen (08) des ersten Zelltyps angeordnet. Es erfolgt ein örtlich gezieltes Anordnen eines zweiten Präkursors (12) der die Zellen (08) verklebenden Substanz (11) auf der Auswahl der primären Zellen (08) des ersten Zelltyps gemäß einer von Zellen des ersten Zelltyps in dem nachzubildenden Gewebe gebildeten Struktur. Dadurch reagieren der erste Präkursor und der zweite Präkursor (12) miteinander und bilden die die Zellen (08) verklebende Substanz (11), welche die Auswahl der primären Zellen (08) des ersten Zelltyps gemäß der von den Zellen des ersten Zelltyps in dem nachzubildenden Gewebe gebildeten Struktur verklebt.

## Beschreibung

Die vorliegende Erfindung betrifft ein Verfahren zum dreidimensionalen Nachbilden eines biologischen Gewebes im Rahmen des Tissue Engineering.

Der Artikel von Murphy, S. V. und Atala, A.: "3D bioprinting of tissues and organs" in Nature Biotechnology, 2014. 32(8), Seiten 773-785 zeigt den Einsatz von 3D-Druckverfahren zur Herstellung von biokompatiblen Materialien, Zellen und unterstützenden Komponenten für komplexe lebendige 3D-Funktionsgewebe.

In dem Artikel von Bassett, D. C.; Hati, A. G.; Melo, T. B.; Stokke, B. T. und Sikorski, P.: "Competitive ligand exchange of crosslinking ions for ionotropic hydrogel formation" in Journal of Material Chemistry B, 2016. 4(37), Seiten 6175-6182 wird ein Ansatz zur Bildung von Hydrogelen aus ionotropen Polymeren durch konkurrierende Verdrängung chelatisierter Ionen beschrieben.

Der Artikel von Hati, A. G.; Bassett, D. C.; Ribe, J. M.; Sikorski, P. et al.: "Versatile, cell and chip friendly method to gel alginate in microfluidic devices" in Lab on a Chip, 2016. 16(19), Seiten 3718-3727 beschreibt die Verwendung von Alginat in mikrofluidischen Geräten zur Herstellung von diskrete Kügelchen oder Fasern im Mikromaßstab. Solche Strukturen können verwendet werden, um empfindliche Zellen und Biomoleküle einzukapseln.

Das Unternehmen ClexBio / Nordovo Biosciences AS, Oslo, Norwegen bietet ein Zweikomponentenhydrogel unter dem Produktnamen "Clex" an.

Die WO 2017/153947 A1 zeigt ein Verfahren zur Bildung eines vernetzten Polymerhydrogels unter Verwendung eines kompetitiven Ligandenaustausches. Bei diesem Verfahren werden eine erste Lösung und eine zweite Lösung vermischt. Die erste Lösung umfasst ein Vernetzungsmittel und einen ersten Chelatbildner. Die zweite Lösung umfasst ein Verdrängungsmittel. Mindestens eine der beiden Lösungen enthält ein ionotropes Polymer.

In dem Artikel von Jeong, H.-J.; Nam, H.; Jang J. und Lee, S.-J.: "3D Bioprinting Strategies for the Regeneration of Functional Tubular Tissues and Organs" in Bioengineering, Basel, 2020, 7, 32, wird die Anwendung der 3D-Bioprinting-Technologie für die Herstellung patientenspezifischer und komplex geformter Freiformarchitekturen beschrieben.

Der Artikel von Gao, Q. Q.; Kim B.-S. und Gao, G.: "Advanced Strategies for 3D Bioprinting of Tissue and Organ Analogs Using Alginate Hydrogel Bioinks" in Marine Drugs, 2021, 19, 708, zeigt die Verwendung des natürlichen Polysaccharids Alginat als Bioink-Quelle für den 3D-Biodruck.

In dem Produktdatenblatt "Printing System Autodrop Compact AD-P-7000" der microdrop Technologies GmbH, Norderstedt, ist ein Drop-on-Demand System mit einem piezogetriebenen Dispenser beschrieben.

Die WO 2011/035937 A1 beschreibt einen mikrostrukturierten Formkörper, umfassend eine Folie, die in unverformte Bereiche und verdünnte Verstreckungsbereiche geteilt ist. Zumindest in einigen der verdünnten Verstreckungsbereiche sind Mikrostrukturen ausgebildet, wobei Poren zumindest in einem der verdünnten Verstreckungsbereiche ausgebildet sind.

Weiterhin ist aus der WO 2011/035938 A1 ein mikrostrukturierter Formkörper bekannt, der einen folienartigen Grundkörper besitzt, welcher eine erste Folienschicht und eine darunter befindliche zweite Folienschicht umfasst, wobei die zweite Folienschicht Ausnehmungen mit einem Durchmesser von weniger als 2 mm aufweist, die durch verformte Bereiche der ersten Folienschicht ausgeformt sind, durch welche Kavitäten ausgebildet sind. Zumindest einige der verformten Bereiche der ersten Folienschicht besitzen Poren.

In der DE 10 2010 037 968 A1 ist eine Struktur zur Nachbildung eines Sinusoids beschrieben, die sich in eine Mikrotiterplatte einsetzen lässt. Die Struktur umfasst mehrere übereinander angeordnete Schichten aus einem porösen Material, wobei zwischen den Schichten jeweils ein Zwischenraum ausgebildet ist. Die Zwischenräume sind durch in den Schichten zur Weiterleitung eines Fluids ausgebildete Kanäle verbunden.

Die DE 10 2015 122 375 A1 betrifft ein Verfahren zum Nachbilden einer Stammzellnische eines Organismus. Es wird ein Abbild eines mindestens eine Stammzellnische umfassenden Gewebes eines Organismus erzeugt. Das Abbild wird gefiltert, um ein Strukturbild der abgebildeten Stammzellnische zu erhalten. In einem weiteren Schritt wird eine lithographische Maske aus dem Strukturbild erzeugt. Es erfolgt ein Strukturieren eines Ausgangsmaterials eines Substrates durch mittelbares oder unmittelbares Anwenden der lithographischen Maske, wodurch ein strukturiertes Substrat erhalten wird, welches die Nachbildung der abgebildeten Stammzellnische des Organismus darstellt.

Ein Überblick über den Einsatz von Hydrogelen zur Nachbildung von Stammzellnischen findet sich in dem Artikel von Gerecht, S. et al.: "Hyaluronic acid hydrogel for controlled selfrenewal and differentiation of human embryonic stem cells" in PNAS, Ausgabe 104, Nr. 27, Seiten 11298-11303, Juli 2007.

In dem Artikel von Chan, V. et al.: "Three-dimensional photopattering of hydrogels using stereolitography for long-term cell encapsulation" in Lab on a Chip, 2010, Ausgabe 10, Seiten 2062-2070, ist ein Verfahren zur Polymerisation von Hydrogelen beschrieben, für welches das Licht eines UV-Lasers genutzt wird. Die polymerisierten Hydrogele bilden Strukturen für Zellen aus.

Die US 2015/0118197 A1 zeigt ein Verfahren zur Herstellung eines elektrogesponnenen Gewebes, durch welches beispielsweise die Morphologie einer Nische in den Vogt-Palisaden eines Limbus nachgebildet werden kann.

Die US 2005/0169962 A1 zeigt ein Verfahren zur Herstellung von strukturierten dreidimensionalen Biopolymergerüsten mit lebenden Zellen. Das Verfahren umfasst die selektive Photopolymerisation von Biopolymeren zur Erzeugung gemusterter Strukturen und die Strukturierung von Zellen in relativ homogenen Biopolymerplatten unter Verwendung der Dielektrophorese.

In dem Artikel von Ye, S. et al.: "Hydrogels for Liver Tissue Engineering" in Bioengineering 2019, 6, 59, doi:10.3390/bioengineering6030059, Seiten 1 bis 30, ist ein Verfahren zum Bioengineering von Lebergewebe beschrieben. Es wird der Einsatz von verschiedenen Hydrogelen erläutert.

Ein Nachteil der aus dem Stand der Technik bekannten Verfahren zur Nachbildung von biologischen Geweben besteht darin, dass die einzubettenden Zellen Beanspruchungen ausgesetzt werden, welche sich negativ auf die Vitalität der Zellen auswirken. Beim 3D-Druck zur Nachbildung von biologischen Geweben werden die Zellen einem hohen Druck und Scherkräften ausgesetzt, um die gewünschte örtliche Auflösung zu erzielen. Schränkt man die örtliche Auflösung ein, so gelingt die Nachbildung des biologischen Gewebes nicht ausreichend. Nutzt man das Prinzip der Photopolymerisation, so werden die Zellen einer schädlichen UV-Strahlung ausgesetzt.

Die Aufgabe der vorliegenden Erfindung besteht ausgehend vom Stand der Technik darin, eine dreidimensionale Nachbildung von biologischen Geweben zu ermöglichen, bei welcher die einzubettenden Zellen keinen für die Zellen schädlichen Beanspruchungen ausgesetzt werden.

Die genannte Aufgabe wird gelöst durch ein Verfahren gemäß dem beigefügten Anspruch 1.

Das erfindungsgemäße Verfahren dient zum dreidimensionalen Nachbilden eines biologischen natürlichen Gewebes. Bei dem biologischen Gewebe handelt es sich bevorzugt um ein biologisches Gewebe eines Organismus. Bei dem Organismus handelt es sich bevorzugt um einen lebenden Organismus, in welchem das Gewebe ausgebildet ist. Bei dem lebenden Organismus handelt es sich insbesondere um ein Tier oder um einen Menschen. Bei dem biologischen Gewebe handelt es sich beispielsweise um ein Gewebe einer Leber eines Menschen oder eines Tieres. Das nachzubildende Gewebe ist beispielsweise durch Lebergewebe gebildet. Im Ergebnis des erfindungsgemäßen Verfahrens wird eine Nachbildung des biologischen Gewebes erhalten, wobei die Nachbildung zumindest einen Teil des biologischen Gewebes dreidimensional nachbildet. Die Nachbildung bildet die Morphologie und die Funktionen des biologischen Gewebes zumindest teilweise nach. Die Nachbildung bildet die Morphologie und die Funktionen des biologischen Gewebes bevorzugt im Wesentlichen vollständig nach. Die Nachbildung bildet bevorzugt einen Teil eines Organes oder ein Organ nach. In diesem Sinne stellt die Nachbildung ein organotypisches oligozelluläres 3-dimensionales Gewebekonstrukt dar.

In einem Schritt des Verfahrens wird ein Substrat bereitgestellt, welches als Basis für den Aufbau der Nachbildung dient. Das Substrat ist bevorzugt biokompatibel. Das Substrat ist bevorzugt eben oder alternativ bevorzugt dreidimensional ausgeformt, um für das dreidimensionale Nachbilden des Gewebes beizutragen.

In einem weiteren Schritt erfolgt ein Vorlegen von primären Zellen eines ersten Zelltyps zumindest auf dem Substrat. Durch das Vorlegen der primären Zellen werden die Zellen auf dem Substrat angeordnet. Die primären Zellen des ersten Zelltyps werden bevorzugt örtlich ungezielt und unstrukturiert auf dem Substrat vorgelegt, sodass sie keine Ordnung aufweisen und in dieser Hinsicht die Morphologie des nachzubildenden Gewebes noch nicht nachbilden. Die primären Zellen des ersten Zelltyps werden bevorzugt gleichmäßig stochastisch verteilt auf dem Substrat vorgelegt bzw. angeordnet. Bevorzugt erfolgt das Vorlegen der primären Zellen durch ein Pipettieren. Die primären Zellen des ersten Zelltyps werden vor deren Vorlegen auf dem Substrat bevorzugt dadurch bereitgestellt, dass sie einer Probe des nachzubildenden Gewebes entnommen werden.

In einem weiteren Schritt erfolgt ein Anordnen eines ersten Präkursors einer später auszubildenden die Zellen verklebenden Substanz auf zumindest einer Auswahl der primären Zellen des ersten Zelltyps. Der erste Präkursors ist ein erster chemischer oder biochemischer Ausgangstoff, welcher benötigt wird, um die die Zellen verklebende Substanz durch eine chemische Reaktion ausbilden zu können. Die auszubildende die Zellen verklebende Substanz wird geeignet sein, diejenigen primären Zellen des ersten Zelltyps, auf denen diese Substanz ausgebildet wird, durch Adhäsionskräfte mechanisch miteinander zu verbinden, wodurch ihre Positionen zueinander und zum Substrat fixiert werden.

In einem weiteren Schritt erfolgt ein örtlich gezieltes Anordnen eines zweiten Präkursors der die Zellen verklebenden Substanz auf der Auswahl der primären Zellen des ersten Zelltyps gemäß einer von Zellen des ersten Zelltyps in dem nachzubildenden natürlichen Gewebe gebildeten Struktur. Dieses Anordnen erfolgt bevorzugt mit einem örtlich dosierenden Ausgabesystem, wie einem in den x-y-Richtungen verfahrbaren Dispenser- oder Pipettiersystem oder bevorzugt einem in den x-y-z-Richtungen verfahrbaren Dispenser- oder Pipettiersystem. Ein Ausgangspunkt für diesen Schritt ist die von den Zellen des ersten Zelltyps in dem nachzubildenden Gewebe gebildete Struktur. Diese Struktur beschreibt die dreidimensionale örtliche Anordnung der Zellen des ersten Zelltyps in dem nachzubildenden Gewebe. Da diese Struktur als Vorlage dazu dient, den zweiten Präkursor der die Zellen verklebenden Substanz auf der Auswahl der primären Zellen des ersten Zelltyps örtlich gezielt anzuordnen, wird eine morphologische Nachbildung des nachzubildenden Gewebes vorgenommen. Durch das örtlich gezielte Anordnen des zweiten Präkursors reagieren der erste Präkursor und der zweite Präkursor an diesen Orten miteinander, sodass sie dort die die Zellen verklebende Substanz bilden, woraufhin diese Substanz die Auswahl der primären Zellen des ersten Zelltyps gemäß der von den Zellen des ersten Zelltyps in dem nachzubildenden Gewebe gebildeten Struktur miteinander verklebt. Dabei wird die Auswahl der primären Zellen des ersten Zelltyps bevorzugt auch gleichzeitig mit dem Substrat verklebt. Nur dort wo der zweite Präkursor örtlich gezielt angeordnet wird, kann er mit dem ersten Präkursor chemisch reagieren und die die Zellen verklebende Substanz ausbilden, sodass nur dort die primären Zellen des ersten Zelltyps verklebt werden. Die nun verklebten primären Zellen des ersten Zelltyps der genannten Auswahl bilden zumindest einen ersten Abschnitt des nachzubildenden biologischen Gewebes nach, sodass sie eine Nachbildung zumindest dieses ersten Abschnittes darstellen. Diese Nachbildung bildet bevorzugt eine erste Schicht der primären Zellen. Die die Struktur bestimmende räumliche Anordnung und Verteilung der Auswahl der primären Zellen wird durch die verklebende Substanz fixiert. Diese räumliche Anordnung und Verteilung sind eine Kopie der räumlichen Anordnung und Verteilung der primären Zellen in dem nachzubildenden natürlichen Gewebe. Die dreidimensionale Struktur der primären Zellen des ersten Zelltyps in dem nachzubildenden natürlichen Gewebe dient als Vorlage, sodass eine Beschreibung dieser Struktur benötigt wird. Insofern diese Beschreibung nicht bereits aus dem Fachwissen bekannt ist, kann das nachzubildende natürliche Gewebe untersucht werden, um eine Beschreibung dieser Struktur zu erhalten.

Ein besonderer Vorteil des erfindungsgemäßen Verfahrens besteht darin, dass die Auswahl der primären Zellen eine örtliche Anordnung gemäß der Struktur des nachzubildenden natürlichen Gewebes erfährt, ohne selbst örtlich gezielt bewegt werden zu müssen. Insbesondere müssen die primären Zellen nicht mit einem örtlich gezielt arbeitenden Dispenser angeordnet werden, durch welchen die primären Zellen hohen Scherkräften ausgesetzt würden, was sich nachteilig auf deren Lebensdauer auswirkt. Ein weiterer Vorteil besteht darin, dass keine Strahlung erforderlich ist, um die die Zellen verklebende Substanz auszubilden, da diese durch die Vereinigung des ersten Präkursor mit dem zweiten Präkursor gebildet wird. Eine solche Strahlung würde sich ebenfalls nachteilig auf die Lebensdauer der Zellen auswirken.

Bei bevorzugten Ausführungsformen weist das bereitzustellende Substrat mikrofluidische Strukturelemente aufweist. Die mikrofluidische Strukturelemente bilden bevorzugt eine mikrofluidische Struktur des nachzubildenden biologischen Gewebes nach. Zumindest sind die mikrofluidische Strukturelemente dazu ausgebildet, mikrofluidische Vorgänge, welche in dem nachzubildenden biologischen Gewebe auftreten, funktionell auch in der Nachbildung zu ermöglichen. Die mikrofluidische Strukturelemente sind bevorzugt durch Öffnungen, Löcher, Gräben, Kanäle und/oder poröse Abschnitte im Substrat gebildet. Im Übrigen weist das Substrat bevorzugt Kavitäten und/oder Erhebungen auf, welche ebenfalls der dreidimensionalen Nachbildung des nachzubildenden biologischen Gewebes dienen.

Das Substrat besteht bevorzugt aus einem Polymer, welches bevorzugt biokompatibel ist. Das Substrat ist bevorzugt durch eine Folie gebildet.

Das Substrat ist bevorzugt durch eine Mikrotiterplatte oder durch einen mikrostrukturierten Formkörper gebildet. Das Substrat ist bevorzugt mindestens 10 mm lang und mindestens 10 mm breit.

Die die Zellen verklebende Substanz ist bevorzugt durch ein Hydrogel gebildet, bei welchem es sich bevorzugt um ein vernetztes Polymerhydrogel handelt. Die die Zellen verklebende Substanz ist bevorzugt durch ein Zweikomponentenhydrogel gebildet. Dabei stellt der erste Präkursor eine erste Komponente des Zweikomponentenhydrogels dar. Der zweite Präkursor stellt eine zweite Komponente des Zweikomponentenhydrogels dar. Der erste Präkursor und der zweite Präkursor liegen bevorzugt jeweils in Form einer Lösung vor. Bei der Bildung der die Zellen verklebenden Substanz aus dem ersten Präkursor und dem zweiten Präkursor erfolgt bevorzugt ein kompetitiver Ligandenaustausch. Die Bildung der die Zellen verklebenden Substanz aus dem ersten Präkursor und dem zweiten Präkursor erfordert eine Zeitdauer, welche insbesondere eine Gelierungszeit darstellt und welche bevorzugt weniger als 1 Sekunde, weiter bevorzugt weniger als 100 ms und besonders bevorzugt weniger als 10 ms beträgt. Die Gelierungszeit ist bevorzugt kleiner als eine Dissipationszeit, damit eine funktionsfähige Fixierung der Zellen des ersten Zelltyps erfolgt. Einer der beiden Präkursoren umfasst bevorzugt ein Vernetzungsmittel und einen ersten Chelatbildner, während der andere der beiden Präkursoren ein Verdrängungsmittel umfasst. Einer der beiden Präkursoren enthält bevorzugt ein ionotropes Polymer. Das ionotrope Polymer ist bevorzugt durch Alginat, Pektin, Poly(galacturonat), Carrageenan, Dextran, Gellan, Sclerogiucan, Chitosan, Polyphosphazen, Natriumpolyacrylat und/oder Polyaminosäure gebildet. Das ionotrope Polymer ist besonders bevorzugt durch Alginat gebildet. Grundsätzlich kann die die Zellen verklebende Substanz auch aus einem anderen Gel oder Klebstoff bestehen.

Bei bevorzugten Ausführungsformen erfolgt das Vorlegen der primären Zellen des ersten Zelltyps in einem Medium; insbesondere in einem flüssigen Medium. Das Medium kann vor dem Vorlegen der primären Zellen des ersten Zelltyps auf dem Substrat angeordnet werden. Das Medium kann aber auch gemeinsam mit den primären Zellen des ersten Zelltyps auf dem Substrat angeordnet werden. Das Anordnen des Mediums auf dem Substrat erfolgt bevorzugt durch ein Pipettieren. Besonders bevorzugt wird jedoch das Substrat im Medium angeordnet und die primären Zellen des ersten Zelltyps werden in das Medium auf das Substrat vorgelegt. Das Medium umfasst bevorzugt zumindest ein Nährmedium für die primären Zellen des ersten Zelltyps, sodass diese im Folgenden genährt werden. Das Medium befindet sich zumindest auf dem Substrat, wobei es bevorzugt zumindest dort angeordnet ist, wo auch die primären Zellen des ersten Zelltyps angeordnet werden.

Bei bevorzugten Ausführungsformen umfasst das Medium den ersten Präkursor der die Zellen verklebenden Substanz oder der erste Präkursor wird in das auf dem Substrat befindliche Medium eingemischt. Bei diesen Ausführungsformen wird der erste Präkursor bevorzugt nicht örtlich gezielt bei einer Auswahl der primären Zellen des ersten Zelltyps angeordnet, sodass sich der erste Präkursor an allen vorgelegten primären Zellen des ersten Zelltyps befindet. Das zum Ausbilden der die Zellen verklebende Substanz beide der Präkursoren erforderlich sind, wird die die Zellen verklebende Substanz ohnehin nur dort gebildet, wo der zweite Präkursor ein örtlich gezielt angeordnet wird. Das Medium umfasst entsprechend bevorzugt das Nährmedium und den ersten Präkursor. Bei alternativ bevorzugten Ausführungsformen erfolgt das Anordnen des ersten Präkursors ebenfalls örtlich gezielt gemäß der von den Zellen des ersten Zelltyps in dem nachzubildenden Gewebe gebildeten Struktur. Dieses Anordnen erfolgt bevorzugt mit einem örtlich dosierenden Ausgabesystem, wie einem in den x-y-Richtungen verfahrbaren Dispenser- oder Pipettiersystem oder bevorzugt einem in den x-y-z-Richtungen verfahrbaren Dispenser- oder Pipettiersystem. Somit werden beide Präkursoren an denselben Orten angeordnet, wo sie die die Zellen verklebende Substanz bilden. Der erste Präkursor und der zweite Präkursor können nacheinander oder gleichzeitig örtlich gezielt angeordnet werden.

Grundsätzlich kann der erste Präkursor vor oder nach dem zweiten Präkursor oder gleichzeitig mit dem zweiten Präkursor an bzw. auf den primären Zellen des ersten Zelltyps angeordnet werden.

Der zweite Präkursor der die Zellen verklebenden Substanz wird bevorzugt mit einem örtlich gezielt arbeitenden Dispenser- oder Pipettiersystem an bzw. auf der Auswahl der primären Zellen des ersten Zelltyps angeordnet. Dabei wird der zweite Präkursor in einzelnen Tropfen an bzw. auf der Auswahl der primären Zellen des ersten Zelltyps angeordnet, wobei die kleinsten dosierbaren Tropfen bevorzugt höchstens 5 Pikoliter des zweiten Präkursors und weiter bevorzugt höchstens 1 Pikoliter des zweiten Präkursors enthalten. Beim örtlich gezielten Anordnen des zweiten Präkursors werden bevorzugt Verfahrwege in alle drei Raumrichtungen zurückgelegt, wobei die Verfahrwege bevorzugt mindestens 10 mm und weiter bevorzugt mindestens 100 mm betragen. Das örtlich gezielte Anordnen erfolgt mit einer Genauigkeit, welche ±100 µm oder besser beträgt. Diese Genauigkeit beträgt weiter bevorzugt ±25 µm oder besser.

Das örtlich gezielt arbeitende Dispensersystem umfasst bevorzugt einen 3D-Drucker mit einem Dispenserkopf, welcher bevorzugt auf einer Inkjet-Technologie beruht. Der Dispenserkopf ist zur örtlich gezielten Ausgabe des zweiten Präkursors ausgebildet. Der Dispenser bildet bevorzugt ein Drop-on-Demand-System. Das örtlich gezielt arbeitende Dispenser- bzw. Pipettiersystem umfasst bevorzugt mehrere Antriebe zum Antreiben des Dispenserkopfes bzw. der Pipette in mehrere Raumrichtungen, um den zweiten Präkursor örtlich gezielt auf der Auswahl der primären Zellen des ersten Zelltyps gemäß der von den Zellen des ersten Zelltyps in dem nachzubildenden Gewebe gebildeten Struktur anzuordnen. Der Dispenserkopf bzw. die Pipette kann bevorzugt in alle drei Raumrichtungen über die oben beschriebenen Verfahrwege verfahren werden.

Nachdem der erste Präkursor und der zweite Präkursor die die Zellen verklebende Substanz gebildet haben, verbleiben diejenigen primären Zellen des ersten Zelltyps, die nicht zur Auswahl gehören, zunächst lose auf dem Substrat, da sie nicht durch die die Zellen verklebende Substanz örtlich fixiert sind. Nur die zu der Auswahl gehörigen primären Zellen des ersten Zelltyps bilden den genannten ersten Abschnitt des nachzubildenden biologischen Gewebes nach, da nur diese primären Zellen gemäß der von den Zellen des ersten Zelltyps in dem nachzubildenden Gewebe gebildeten Struktur angeordnet sind. Die übrigen primären Zellen sind außerhalb dieser Struktur lose angeordnet und werden jedenfalls in dieser Verfahrensphase nicht mehr benötigt, sodass sie bevorzugt vom Substrat entfernt werden, was beispielsweise durch einen Waschvorgang erfolgen kann. Bevorzugt erfolgt das Entfernen der übrigen primären Zellen durch ein Pipettieren. Bei denjenigen Ausführungsformen, bei denen der erste Präkursor nicht örtlich gezielt angeordnet wurde, verbleiben auch Reste des ersten Präkursors an denjenigen primären Zellen des ersten Zelltyps, die nicht zur Auswahl gehören. Diese Reste des ersten Präkursors werden jedenfalls in dieser Verfahrensphase nicht mehr benötigt, sodass sie bevorzugt gemeinsam mit den verbliebenden losen primären Zellen des ersten Zelltyps vom Substrat entfernt werden. Die entfernten primären Zellen des ersten Zelltyps und die entfernten Reste des ersten Präkursors werden aber bevorzugt für spätere Schritte des Verfahrens wiederverwendet.

Bei bevorzugten Ausführungsformen wird zusätzlich zu dem das Gewebe nachbildenden ersten Abschnitt mindestens ein weiterer Abschnitt des Gewebes nachgebildet. Die Nachbildung des weiteren Abschnittes kann durch weitere primäre Zellen des ersten Zelltyps oder durch primäre Zellen eines zweiten Zelltyps zur Schaffung einer Kokultur gebildet sein. Im ersten Fall werden hierzu die Schritte des Vorlegens von primären Zellen des ersten Zelltyps, des Anordnens des ersten Präkursors und des örtlich gezielten Anordnens des zweiten Präkursors wiederholt. Somit erfolgt zunächst ein Vorlegen bzw. Anordnen weiterer primärer Zellen des ersten Zelltyps auf der bereits ausgebildeten Nachbildung des ersten Abschnittes des biologischen Gewebes. Der erste Präkursor der die Zellen verklebenden Substanz wird an bzw. auf zumindest einer Auswahl der weiteren primären Zellen des ersten Zelltyps angeordnet. Es erfolgt ein örtlich gezieltes Anordnen des zweiten Präkursors der die Zellen verklebenden Substanz auf bzw. an der Auswahl der weiteren primären Zellen des ersten Zelltyps gemäß der von Zellen des ersten Zelltyps in dem nachzubildenden Gewebe gebildeten Struktur. Der erste Präkursor und der zweite Präkursor werden vereinigt und reagieren chemisch miteinander, sodass sie die die Zellen verklebende Substanz bilden, welche die Auswahl der weiteren primären Zellen des ersten Zelltyps gemäß der von Zellen des ersten Zelltyps in dem nachzubildenden Gewebe gebildeten Struktur verklebt, sodass die Auswahl der weiteren primären Zellen des ersten Zelltyps den zweiten Abschnitt des nachzubildenden biologischen Gewebes nachbildet, wodurch sie eine Nachbildung dieses weiteren Abschnittes darstellen. Dabei wird die Auswahl der weiteren primären Zellen des ersten Zelltyps gleichzeitig zumindest teilweise mit der Nachbildung des ersten Abschnittes des biologischen Gewebes verklebt. Die Nachbildung des weiteren Abschnittes des nachzubildenden biologischen Gewebes bildet bevorzugt eine zweite Schicht der primären Zellen.

Bei Ausführungsformen, bei denen die Nachbildung des weiteren Abschnittes durch primäre Zellen des zweiten Zelltyps gebildet werden soll, erfolgt zunächst ein Vorlegen bzw. Anordnen von primären Zellen des zweiten Zelltyps auf dem Substrat und/oder auf der bereits ausgebildeten Nachbildung des ersten Abschnittes des biologischen Gewebes. Der erste Präkursor der die Zellen verklebenden Substanz wird an bzw. auf zumindest einer Auswahl der primären Zellen des zweiten Zelltyps angeordnet. Es erfolgt ein örtlich gezieltes Anordnen des zweiten Präkursors der die Zellen verklebenden Substanz auf bzw. an der Auswahl der primären Zellen des zweiten Zelltyps gemäß einer von Zellen des zweiten Zelltyps in dem nachzubildenden Gewebe gebildeten Struktur. Der erste Präkursor und der zweite Präkursor werden vereinigt und reagieren chemisch miteinander, sodass sie die die Zellen verklebende Substanz bilden, welche die Auswahl der primären Zellen des zweiten Zelltyps gemäß der von Zellen des zweiten Zelltyps in dem nachzubildenden Gewebe gebildeten Struktur verklebt, sodass die Auswahl der primären Zellen des zweiten Zelltyps den zweiten Abschnitt des nachzubildenden biologischen Gewebes nachbildet, wodurch sie eine Nachbildung dieses weiteren Abschnittes darstellen. Dabei wird die Auswahl der primären Zellen des zweiten Zelltyps gleichzeitig mit dem Substrat und/oder zumindest teilweise mit der Nachbildung des ersten Abschnittes des biologischen Gewebes verklebt. Die Nachbildung des weiteren Abschnittes des nachzubildenden biologischen Gewebes bildet bevorzugt eine zweite Schicht von primären Zellen bzw. eine erste Schicht der primären Zellen des zweiten Zelltyps.

Die Schritte des Vorlegens der weiteren primären Zellen des ersten Zelltyps bzw. der primärer Zellen des zweiten Zelltyps, des anschließenden Anordnens des ersten Präkursors und des örtlich gezielten Anordnens des zweiten Präkursors erfolgen bevorzugt gemäß den oben beschriebenen bevorzugten Ausführungsformen betreffend das Vorlegen der primären Zellen des ersten Zelltyps und dem anschließenden Anordnen des ersten Präkursors und das örtlich gezielte Anordnens des zweiten Präkursors.

Bei weiteren bevorzugten Ausführungsformen werden noch weitere Abschnitte des Gewebes nachgebildet. Für die Nachbildungen der weiteren Abschnitte werden bevorzugt auch primäre Zellen eines dritten und/oder eines weiteren Zelltyps verwendet.

Bei bevorzugten Ausführungsformen werden mindestens fünf der Abschnitte des biologischen Gewebes nachgebildet, sodass die Nachbildung mindestens fünf der Schichten von primären Zellen von einem oder mehreren Zelltypen umfasst.

Eine Nachbildung eines biologischen Gewebes ist durch das erfindungsgemäße Verfahren herstellbar. Die Nachbildung ist bevorzugt durch eine der beschriebenen bevorzugten Ausführungsformen des erfindungsgemäßen Verfahrens herstellbar. Im Übrigen weist die Nachbildung bevorzugt auch solche Merkmale auf, die im Zusammenhang mit dem erfindungsgemäßen Verfahren beschrieben sind.

Weitere Vorteile, Einzelheiten und Weiterbildungen der Erfindung ergeben sich aus der nachfolgenden Beschreibung bevorzugter Ausführungsformen der Erfindung, unter Bezugnahme auf die Zeichnung. Es zeigen:
- Fig. 1:: eine erste Phase einer ersten bevorzugten Ausführungsform eines erfindungsgemäßen Verfahrens zum Nachbilden von biologischem Gewebe;
- Fig. 2:: eine zweite Phase der ersten bevorzugten Ausführungsform des erfindungsgemäßen Verfahrens;
- Fig. 3:: eine dritte Phase der ersten bevorzugten Ausführungsform des erfindungsgemäßen Verfahrens;
- Fig. 4:: eine vierte Phase der ersten bevorzugten Ausführungsform des erfindungsgemäßen Verfahrens;
- Fig. 5:: eine fünfte Phase der ersten bevorzugten Ausführungsform des erfindungsgemäßen Verfahrens;
- Fig. 6:: eine sechste Phase der ersten bevorzugten Ausführungsform des erfindungsgemäßen Verfahrens;
- Fig. 7:: eine erste Phase einer zweiten bevorzugten Ausführungsform des erfindungsgemäßen Verfahrens zum Nachbilden von biologischem Gewebe;
- Fig. 8:: eine zweite Phase der zweiten bevorzugten Ausführungsform des erfindungsgemäßen Verfahrens;
- Fig. 9:: eine dritte Phase der zweiten bevorzugten Ausführungsform des erfindungsgemäßen Verfahrens;
- Fig. 10:: eine vierte Phase der zweiten bevorzugten Ausführungsform des erfindungsgemäßen Verfahrens;
- Fig. 11:: eine fünfte Phase der zweiten bevorzugten Ausführungsform des erfindungsgemäßen Verfahrens; und
- Fig. 12:: eine sechste Phase der zweiten bevorzugten Ausführungsform des erfindungsgemäßen Verfahrens.

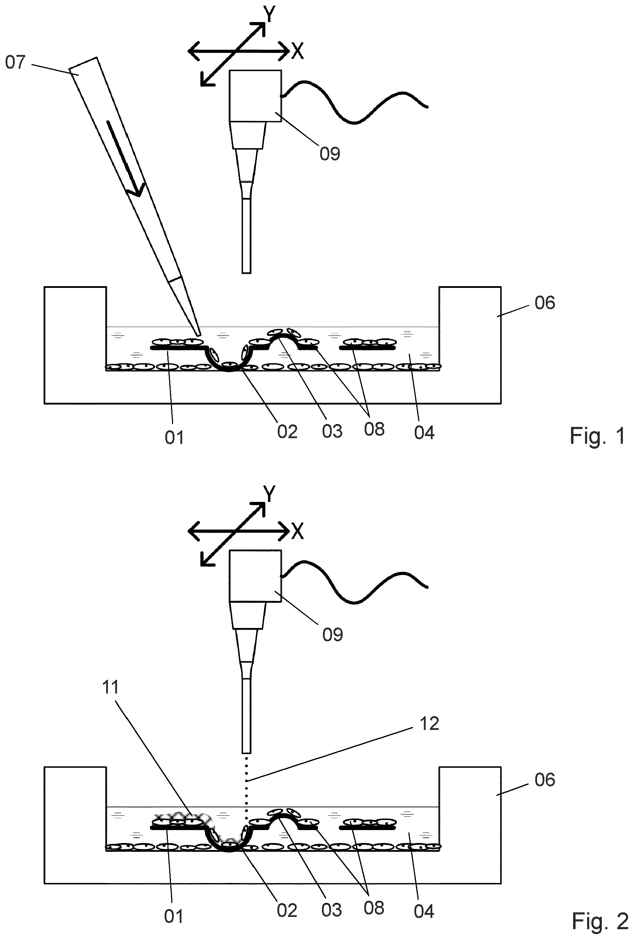

Fig. 1 zeigt eine erste Phase einer ersten bevorzugten Ausführungsform eines erfindungsgemäßen Verfahrens zum Nachbilden von biologischem Gewebe. Ein strukturiertes Substrat 01 in Form einer Folie mit Kavitäten 02 und Erhebungen 03 wird bereitgestellt und in einem Medium 04 in einer Bearbeitungsschale 06 angeordnet. Mit einer Pipette 07 werden primäre Zellen 08 eines ersten Zelltyps auf dem Substrat 01 vorgelegt. Dabei kommt es dazu, dass einige der primären Zellen 08 des ersten Zelltyps nicht auf dem Substrat 01 verbleiben sondern auf einen Boden der Bearbeitungsschale 06 gelangen. Die primäre Zellen 08 des ersten Zelltyps werden mit der Pipette 07 örtlich ungezielt über das Substrat 01 verteilt, sodass sich die primären Zellen 08 weitgehend gleichmäßig verteilen. Beim Vorlegen der primären Zellen 08 des ersten Zelltyps mit der Pipette 07 werden die primären Zellen 08 keinem hohen Druck und keiner Scherbeanspruchung ausgesetzt. Über der Bearbeitungsschale 06 ist ein in dieser ersten Phase noch nicht genutztes in x-y-Richtungen verfahrbares Dispensersystem 09 angeordnet. Das Dispensersystem 09 kann bevorzugt auch zum Verfahren in die drei x-y-z-Richtungen ausgebildet sein oder auch durch ein Pipettiersystem gebildet sein.

Das Medium 04 umfasst einen ersten Präkursor für eine später auszubildende die primären Zellen 08 verklebende Substanz 11 (gezeigt in Fig. 2). Das Medium 04 umfasst zudem ein Nährmedium für die primären Zellen 08 des ersten Zelltyps.

Fig. 2 zeigt eine zweite Phase der ersten bevorzugten Ausführungsform des erfindungsgemäßen Verfahrens zum Nachbilden von biologischem Gewebe. In dieser zweiten Phase wird das in die x-y-Richtungen verfahrbare Dispensersystem 09 genutzt, um eine zweiten Präkursor 12 der die Zellen 08 verklebenden Substanz 11 auf einer Auswahl der primären Zellen 08 des ersten Zelltyps örtlich gezielt anzuordnen. Der zweite Präkursor 12 wird gemäß einer Struktur örtlich gezielt angeordnet, wie sie von den Zellen 08 des ersten Zelltyps in dem nachzubildenden Gewebe (nicht gezeigt) tatsächlich gebildet wird. Der im Medium 04 vorhandene erste Präkursor und der zweite Präkursor 12 bilden sofort die die Zellen 08 verklebende Substanz 11 in Form eines Hydrogels. Die verklebte Auswahl der primären Zellen 08 des ersten Zelltyps stellt eine Nachbildung eines ersten Abschnittes des nachzubildenden biologischen Gewebes (nicht gezeigt) dar.

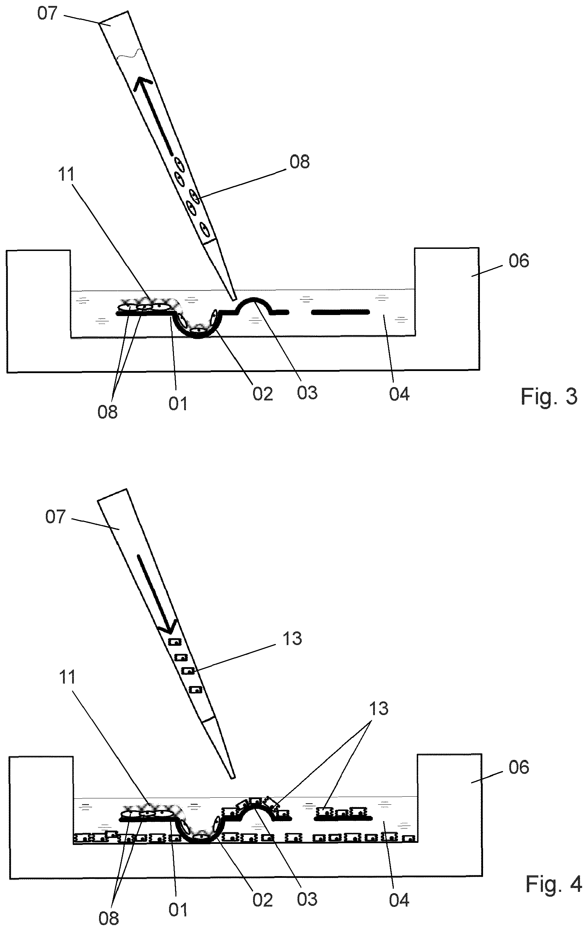

Fig. 3 zeigt eine dritte Phase der ersten bevorzugten Ausführungsform des erfindungsgemäßen Verfahrens zum Nachbilden von biologischem Gewebe. In dieser dritten Phase werden die nicht durch die Substanz 11 verklebten primären Zellen 08 des ersten Zelltyps mit der Pipette 07 vom Substrat 01 und aus dem Medium 04 entfernt.

Fig. 4 zeigt eine vierte Phase der ersten bevorzugten Ausführungsform des erfindungsgemäßen Verfahrens zum Nachbilden von biologischem Gewebe. Mit der Pipette 07 werden nun primäre Zellen 13 eines zweiten Zelltyps auf dem Substrat 01 vorgelegt. Dabei kommt es dazu, dass einige der primären Zellen 13 des zweiten Zelltyps nicht auf dem Substrat 01 verbleiben sondern auf den Boden der Bearbeitungsschale 06 gelangen. Die primäre Zellen 13 des zweiten Zelltyps werden mit der Pipette 07 örtlich ungezielt über das Substrat 01 verteilt, sodass sich die primären Zellen 13 weitgehend gleichmäßig verteilen. Beim Vorlegen der primären Zellen 13 des zweiten Zelltyps mit der Pipette 07 werden die primären Zellen 13 keinem hohen Druck und keiner Scherbeanspruchung ausgesetzt.

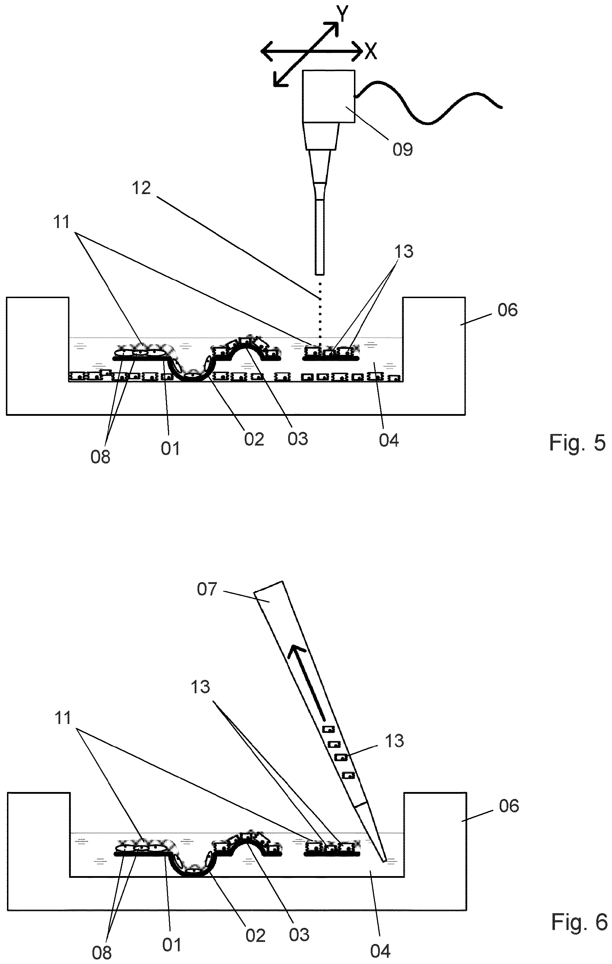

Fig. 5 zeigt eine fünfte Phase der ersten bevorzugten Ausführungsform des erfindungsgemäßen Verfahrens zum Nachbilden von biologischem Gewebe. In dieser fünften Phase wird das in die x-y-Richtungen verfahrbare Dispensersystem 09 genutzt, um weitere Mengen des zweiten Präkursors 12 der die Zellen 08, 13 verklebenden Substanz 11 auf einer Auswahl der primären Zellen 13 des zweiten Zelltyps örtlich gezielt anzuordnen. Der zweite Präkursor 12 wird gemäß einer Struktur örtlich gezielt angeordnet, wie sie von den Zellen 13 des zweiten Zelltyps in dem nachzubildenden Gewebe (nicht gezeigt) tatsächlich gebildet wird. Der im Medium 04 vorhandene erste Präkursor und der zweite Präkursor 12 bilden wiederum sofort die die Zellen 08, 13 verklebende Substanz 11 in Form eines Hydrogels. Die verklebte Auswahl der primären Zellen 13 des zweiten Zelltyps stellt eine Nachbildung eines zweiten Abschnittes des nachzubildenden biologischen Gewebes (nicht gezeigt) dar.

Fig. 6 zeigt eine sechste Phase der ersten bevorzugten Ausführungsform des erfindungsgemäßen Verfahrens zum Nachbilden von biologischem Gewebe. In dieser sechsten Phase werden die nicht durch die Substanz 11 verklebten primären Zellen 13 des zweiten Zelltyps mit der Pipette 07 vom Substrat 01 und aus dem Medium 04 entfernt.

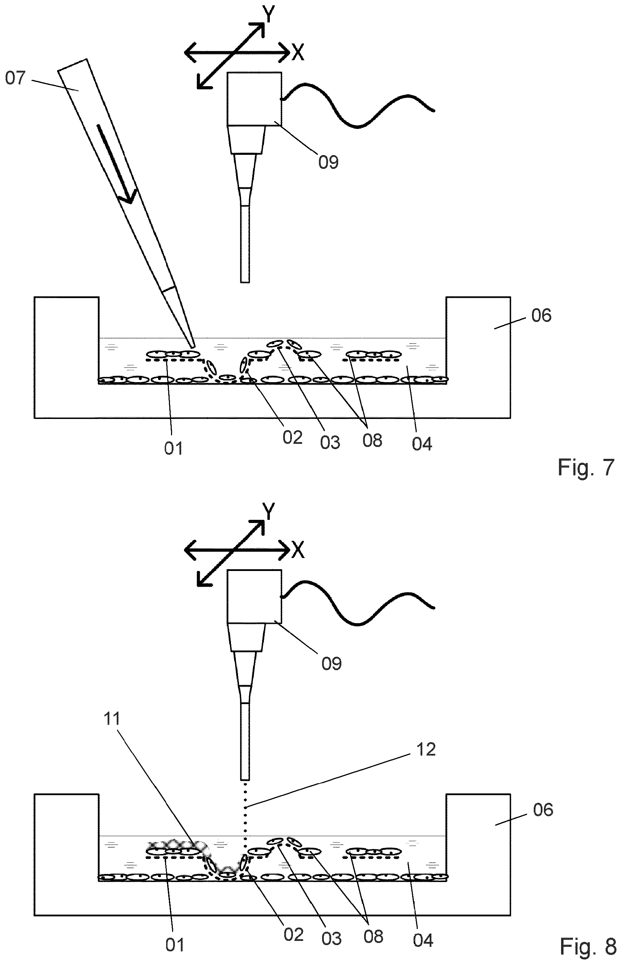

Fig. 7 zeigt eine erste Phase einer zweiten bevorzugten Ausführungsform des erfindungsgemäßen Verfahrens zum Nachbilden von biologischem Gewebe. Diese zweite Ausführungsform gleicht zunächst der in den Figuren 1 bis 6 veranschaulichten ersten Ausführungsform. Im Unterschied zur ersten Ausführungsform ist bei der zweiten Ausführungsform das Substrat 01 porös ausgebildet. Der Ablauf der ersten Phase der zweiten Ausführungsform gleicht dem in Fig. 1 gezeigten Ablauf der ersten Phase der ersten Ausführungsform.

Fig. 8 zeigt eine zweite Phase der zweiten bevorzugten Ausführungsform des erfindungsgemäßen Verfahrens zum Nachbilden von biologischem Gewebe. Der Ablauf der zweiten Phase der zweiten Ausführungsform gleicht dem in Fig. 2 gezeigten Ablauf der zweiten Phase der ersten Ausführungsform.

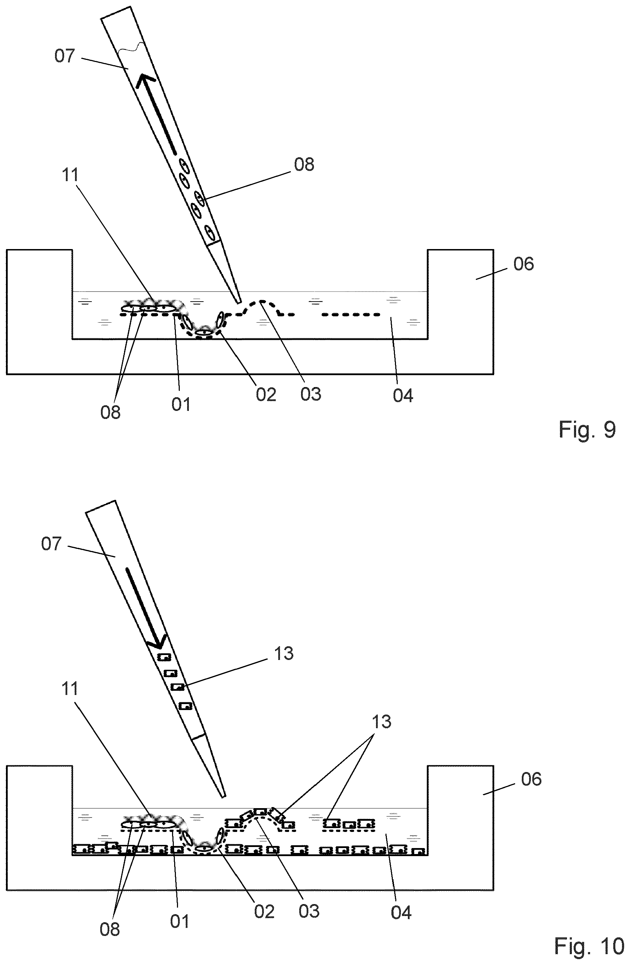

Fig. 9 zeigt eine dritte Phase der zweiten bevorzugten Ausführungsform des erfindungsgemäßen Verfahrens zum Nachbilden von biologischem Gewebe. Der Ablauf der dritten Phase der zweiten Ausführungsform gleicht dem in Fig. 3 gezeigten Ablauf der dritten Phase der ersten Ausführungsform.

Fig. 10 zeigt eine vierte Phase der zweiten bevorzugten Ausführungsform des erfindungsgemäßen Verfahrens zum Nachbilden von biologischem Gewebe. Der Ablauf der vierten Phase der zweiten Ausführungsform gleicht dem in Fig. 4 gezeigten Ablauf der vierten Phase der ersten Ausführungsform.

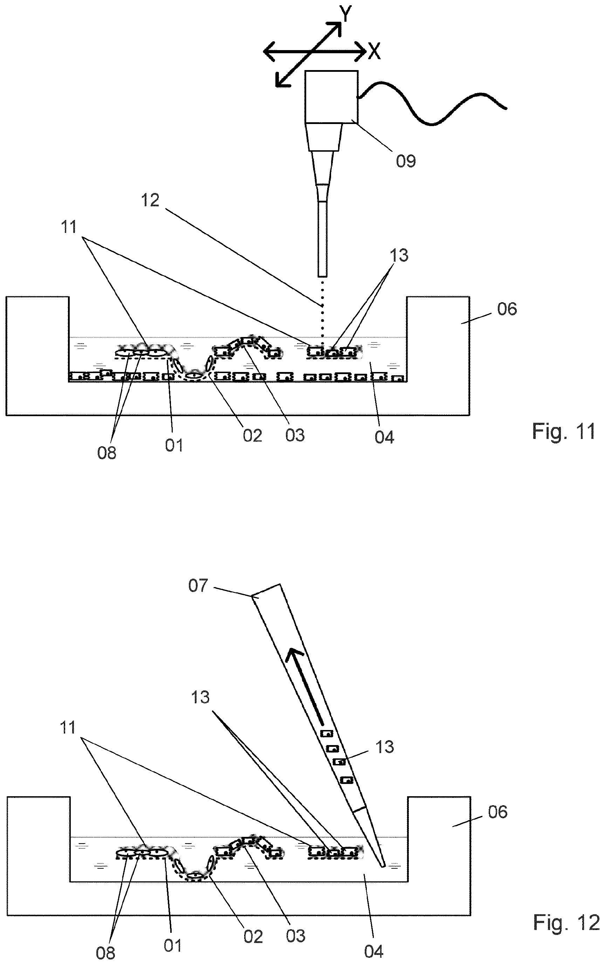

Fig. 11 zeigt eine fünfte Phase der zweiten bevorzugten Ausführungsform des erfindungsgemäßen Verfahrens zum Nachbilden von biologischem Gewebe. Der Ablauf der fünften Phase der zweiten Ausführungsform gleicht dem in Fig. 5 gezeigten Ablauf der fünften Phase der ersten Ausführungsform.

Fig. 12 zeigt eine sechste Phase der zweiten bevorzugten Ausführungsform des erfindungsgemäßen Verfahrens zum Nachbilden von biologischem Gewebe. Der Ablauf der sechsten Phase der zweiten Ausführungsform gleicht dem in Fig. 6 gezeigten Ablauf der sechsten Phase der ersten Ausführungsform.

### Bezugszeichenliste

- 01: Substrat
- 02: Kavität
- 03: Erhebung
- 04: Medium
- 05: -
- 06: Bearbeitungsschale
- 07: Pipette
- 08: primäre Zellen eines ersten Zelltyps
- 09: verfahrbares Dispensersystem
- 10: -
- 11: die primären Zellen verklebende Substanz
- 12: zweiter Präkursor
- 13: primäre Zellen eines zweiten Zelltyps

## Patentansprüche

1. Verfahren zum Nachbilden von biologischem Gewebe, folgende Schritte umfassend:
- Bereitstellen eines Substrates (01);
- Vorlegen von primären Zellen (08) eines ersten Zelltyps auf dem Substrat (01);
- Anordnen eines ersten Präkursors einer die Zellen (08, 13) verklebenden Substanz (11) auf zumindest einer Auswahl der primären Zellen (08) des ersten Zelltyps; und
- örtlich gezieltes Anordnen eines zweiten Präkursors (12) der die Zellen (08, 13) verklebenden Substanz (11) auf der Auswahl der primären Zellen (08) des ersten Zelltyps gemäß einer von Zellen des ersten Zelltyps in dem nachzubildenden Gewebe gebildeten Struktur, wodurch der erste Präkursor und der zweite Präkursor (12) miteinander reagieren und die die Zellen (08, 13) verklebende Substanz (11) bilden, welche die Auswahl der primären Zellen (08) des ersten Zelltyps gemäß der von den Zellen des ersten Zelltyps in dem nachzubildenden Gewebe gebildeten Struktur verklebt und eine erste Schicht der primären Zellen (08) bildet, sodass die Auswahl der primären Zellen (08) des ersten Zelltyps eine Nachbildung zumindest eines ersten Abschnittes des nachzubildenden biologischen Gewebes darstellt.

2. Verfahren nach Anspruch 1, **dadurch gekennzeichnet, dass** das bereitzustellende Substrat (01) mikrofluidische Strukturelemente aufweist.

3. Verfahren nach Anspruch 1 oder 2, **dadurch gekennzeichnet, dass** die primären Zellen (08) des ersten Zelltyps gleichmäßig stochastisch verteilt auf dem Substrat (01) vorgelegt werden.

4. Verfahren nach einem der Ansprüche 1 bis 3, **dadurch gekennzeichnet, dass** das Anordnen des ersten Präkursors der die Zellen (08, 13) verklebenden Substanz (11) auf der Auswahl der primären Zellen (08) des ersten Zelltyps mit einem örtlich dosierenden Ausgabesystem (09) erfolgt.

5. Verfahren nach Anspruch 4, **dadurch gekennzeichnet, dass** das örtlich dosierende Ausgabesystem (09) durch ein in den x-y-z-Richtungen verfahrbares Dispenser- oder Pipettiersystem gebildet ist.

6. Verfahren nach Anspruch 4 oder 5, **dadurch gekennzeichnet, dass** der zweite Präkursor (12) in einzelnen Tropfen an oder auf der Auswahl der primären Zellen (08) des ersten Zelltyps angeordnet wird, wobei die kleinsten dosierbaren Tropfen höchstens 5 Pikoliter des zweiten Präkursors (12) enthalten.

7. Verfahren nach einem der Ansprüche 4 bis 6, **dadurch gekennzeichnet, dass** beim örtlich gezielten Anordnen des zweiten Präkursors (12) mit dem örtlich dosierenden Ausgabesystem (09) Verfahrwege in alle drei Raumrichtungen zurückgelegt werden, wobei die Verfahrwege mindestens 10 mm betragen.

8. Verfahren nach einem der Ansprüche 1 bis 7, **dadurch gekennzeichnet, dass** die die Zellen (08, 13) verklebende Substanz (11) durch ein Zweikomponentenhydrogel gebildet ist, wobei der erste Präkursor eine erste Komponente des Zweikomponentenhydrogels darstellt und der zweite Präkursor (12) eine zweite Komponente des Zweikomponentenhydrogels darstellt.

9. Verfahren nach einem der Ansprüche 1 bis 8, **dadurch gekennzeichnet, dass** einer der beiden Präkursoren (12) ein ionotropes Polymer enthält, welches durch Alginat, Pektin, Poly(galacturonat), Carrageenan, Dextran, Gellan, Sclerogiucan, Chitosan, Polyphosphazen, Natriumpolyacrylat und/oder Polyaminosäure gebildet ist.

10. Verfahren nach einem der Ansprüche 1 bis 9, **dadurch gekennzeichnet, dass** das Vorlegen der primären Zellen (08) des ersten Zelltyps in einem Medium (04) erfolgt.

11. Verfahren nach Anspruch 10, **dadurch gekennzeichnet, dass** das Medium (04) zumindest ein Nährmedium für die primären Zellen (08) des ersten Zelltyps umfasst.

12. Verfahren nach Anspruch 10 oder 11, **dadurch gekennzeichnet, dass** das Medium (04) weiterhin den ersten Präkursor der die Zellen (08, 13) verklebenden Substanz (11) umfasst, oder dass der erste Präkursor der die Zellen (08, 13) verklebenden Substanz (11) in das auf dem Substrat (01) befindliche Medium (04) eingemischt wird.

13. Verfahren nach einem der Ansprüche 1 bis 12, **dadurch gekennzeichnet, dass**, nachdem der erste Präkursor und der zweite Präkursor (12) die die Zellen (08, 13) verklebende Substanz (11) gebildet haben, die nicht zu der Auswahl gehörigen primären Zellen (08) des ersten Zelltyps vom Substrat (01) entfernt werden.

14. Verfahren nach einem der Ansprüche 1 bis 13, **dadurch gekennzeichnet, dass** es folgende weitere Schritte umfasst:
- Vorlegen weiterer primärer Zellen (08) des ersten Zelltyps auf der bereits ausgebildeten Nachbildung des ersten Abschnittes des biologischen Gewebes;
- Anordnen des ersten Präkursors der die Zellen (08, 13) verklebenden Substanz (11) auf zumindest einer Auswahl der weiteren primären Zellen (08) des ersten Zelltyps; und
- örtlich gezieltes Anordnen des zweiten Präkursors (12) der die Zellen (08, 13) verklebenden Substanz (11) auf der Auswahl der weiteren primären Zellen (08) des ersten Zelltyps gemäß der von Zellen des ersten Zelltyps in dem nachzubildenden Gewebe gebildeten Struktur, wodurch der erste Präkursor und der zweite Präkursor (12) miteinander reagieren und die die Zellen (08, 13) verklebende Substanz (11) bilden, welche die Auswahl der weiteren primären Zellen (08) des ersten Zelltyps gemäß der von den Zellen des ersten Zelltyps in dem nachzubildenden Gewebe gebildeten Struktur verklebt, sodass die Auswahl der weiteren primären Zellen (08) des ersten Zelltyps eine Nachbildung eines weiteren Abschnittes des nachzubildenden biologischen Gewebes darstellt.

15. Verfahren nach einem der Ansprüche 1 bis 14, **dadurch gekennzeichnet, dass** es folgende weitere Schritte umfasst:
- Vorlegen von primären Zellen (13) eines zweiten Zelltyps auf dem Substrat (01) und/oder auf der bereits ausgebildeten Nachbildung des ersten Abschnittes des biologischen Gewebes;
- Anordnen des ersten Präkursors der die Zellen (08, 13) verklebenden Substanz (11) auf zumindest einer Auswahl der primären Zellen (13) des zweiten Zelltyps; und
- örtlich gezieltes Anordnen des zweiten Präkursors (12) der die Zellen (08, 13) verklebenden Substanz (11) auf der Auswahl der primären Zellen (13) des zweiten Zelltyps gemäß einer von Zellen des zweiten Zelltyps in dem nachzubildenden Gewebe gebildeten Struktur, wodurch der erste Präkursor und der zweite Präkursor (12) vereinigt werden und die die Zellen (08, 13) verklebende Substanz (11) bilden, welche die Auswahl der primären Zellen (13) des zweiten Zelltyps gemäß der von den Zellen des zweiten Zelltyps in dem nachzubildenden Gewebe gebildeten Struktur verklebt, sodass die Auswahl der primären Zellen (13) des zweiten Zelltyps eine Nachbildung eines weiteren Abschnittes des nachzubildenden biologischen Gewebes darstellt.
